# EUROPÄISCHE PATENTANMELDUNG

(11) **EP 4 826 730 A1**
(43) Veröffentlichungstag der Anmeldung: **21.10.2026**
(21) Anmeldenummer: 26171066.9
(22) Anmeldetag: 07.04.2026
(51) Int. Cl.: G16H 20/17, G16H 40/63, G16H 40/67

(54) **MEDIZINISCHE PUMPE MIT KONTAKTLOSER DATENÜBERTRAGUNG**

(30) Priorität: 14.04.2025 EP 25170456
(71) Anmelder: B. Braun Melsungen AG, 34212 Melsungen (DE)
(72) Erfinder: LANDMESSER, David, 34128 Kassel (DE); DISCHER, Markus, 34593 Knüllwald (DE)
(74) Vertreter: Tergau & Walkenhorst Intellectual Property GmbH

(57) **Zusammenfassung**

Medizinischen Pumpe (1) umfassend eine Komponente (2) zur kontaktlosen Datenübertragung von Pumpendaten an ein mobiles Endgerät (3), wobei die Komponente (2) die Pumpendaten umfasst, und
wobei die Pumpendaten in einem für die Übertragung oder Speicherung geeigneten Datenpaket für die Verarbeitung oder Analyse durch nachgelagerte Systeme oder Anwendungen, insbesondere eine Serviceeinrichtung (4), strukturiert sind.

## Beschreibung

### Technisches Gebiet

Die vorliegende Erfindung betrifft eine medizinische Pumpe, ein Verfahren zur kabellosen Übertragung von Pumpendaten, eine App und ein System zur kabellosen Übertragung von Pumpendaten.

### Hintergrund

Im Bereich der Medizintechnik sind medizinische Pumpen, beispielsweise Infusionspumpen oder Spritzenpumpen, weit verbreitet und spielen eine entscheidende Rolle bei der Verabreichung von Medikamenten und anderen medizinischen Flüssigkeiten an Patienten. Der aktuelle Stand der Technik umfasst verschiedene Arten von Pumpen, die in der Lage sind, präzise Dosierungen zu liefern und dabei eine hohe Zuverlässigkeit zu gewährleisten. Diese Pumpen sind oft mit verschiedenen Sensoren und Überwachungssystemen ausgestattet, um ihre Funktionalität und Sicherheit zu erhöhen.

Dabei besteht das Problem, dass trotz der Fortschritte in der Technologie immer noch erhebliche Herausforderungen im Umgang mit medizinischen Pumpen existieren. Ein zentrales Problem ist die lange Dauer der Auswertung von Fehlern. Wenn eine Pumpe ausfällt oder Fehlfunktionen aufweist, kann es viel Zeit in Anspruch nehmen, die genaue Ursache zu identifizieren und zu beheben.

Im Stand der Technik sind dabei zwar Systeme bekannt, bei denen Daten vernetzter medizinischer Pumpen über online Applikationen ausgelesen werden können. Das Auslesen über solche online Applikationen ist allerdings nur geschulten Medizintechnikern bzw. Mitarbeitern der Pumpenherstellern erlaubt und kann insbesondere über einen Fernzugriff, bspw. via VPN, erfolgen. Damit besteht auch das Problem, dass aus Sicherheitsgründen, medizinischem Personal ohne entsprechende Ausbildung oder Schulung der Zugriff auf die entsprechende Software nicht erlaubt ist.

Insbesondere bei Pumpen, die nicht in ein Netzwerk eingebunden sind, ist somit das Auslesen von Daten schwierig. Dies ist zum einen noch bei vielen Krankenhäusern weltweit der Fall. Zum anderen sind medizinische Pumpen auch in nichtvernetztem Einsatz bspw. im Transport (Krankenwagen, Helikopter), in Arztpraxen oder Tageskliniken.

In derartigen Szenarien, müssen die Fehlerdaten manuell ausgelesen und analysiert werden, was zeitaufwendig und fehleranfällig sein kann. Die Pumpe wird häufig ausgebaut und an entsprechende Servicestellen, insbesondere die Hersteller, gesandt, um dort zunächst analysiert zu werden.

Dies führt zu einer erhöhten Arbeitsbelastung für das Klinikpersonal. Darüber hinaus können unzureichende oder verspätete Fehleranalysen das Risiko von wiederkehrenden Problemen erhöhen, was die Zuverlässigkeit der Pumpen ebenfalls beeinträchtigt.

Das Klinikpersonal steht dabei vor der Herausforderung, die im Betrieb anfallenden technischen Probleme und Störungen schnell und effizient zu lösen. Die Notwendigkeit, Fehlerdaten manuell auszulesen und zu analysieren, erhöht dabei die Arbeitsbelastung, die auch dadurch erhöht wird, dass die manuelle Analyse oft spezielles technisches Wissen erfordert, welches nicht immer sofort verfügbar ist oder das vorhandene Personal überfordert.

In der Praxis besteht ein weiterer Aufwand insbesondere im Rahmen wiederkehrender sicherheitstechnischer Kontrollen (STK) bzw. periodischer Wartungs- und Prüfprozesse medizinischer Pumpen. Hierbei müssen häufig eine Vielzahl von Pumpen identifiziert und dokumentiert werden, wobei Geräteinformationen wie Seriennummer, Herstellungsdatum und Pumpentyp typischerweise manuell vom Typenschild abgelesen und in Systeme der Medizintechnik übertragen werden. Zudem kann zur Kennzeichnung des Prüfstatus (z. B. "STK durchgeführt") eine manuelle, visuelle Markierung am Gerät (beispielsweise ein Farbpunkt) verwendet werden.

Die manuelle Erfassung dieser Geräteinformationen ist zeitaufwendig und fehleranfällig und bindet geschultes Personal. Ferner besteht ein Bedarf, Prüfstatus- oder Wartungsinformationen unmittelbar am Gerät effizient zu prüfen und zu dokumentieren, auch wenn die Pumpe nicht in ein Netzwerk eingebunden ist und ohne dass hierfür zwingend ein Ausbau oder Transport des Geräts erforderlich wird.

### Zusammenfassende Beschreibung der Erfindung

Es ist deshalb eine der vorliegenden Erfindung zugrunde liegende Aufgabe, die Nachteile von medizinischen Pumpen im Stand der Technik zu überwinden. Insbesondere ist es eine der vorliegenden Erfindung zugrunde liegende Aufgabe, medizinische Pumpen und Systeme bereitzustellen, die über eine verbesserte Fehlerauswertung und Datenübertragung verfügen.

Diese und andere Probleme werden durch den Gegenstand der beigefügten unabhängigen Ansprüche gelöst.

Bevorzugte Ausführungsformen können den abhängigen Ansprüchen entnommen werden und darüber hinaus aus der folgenden Beschreibung, insbesondere unter Berücksichtigung verschiedener Ausführungsformen, wie sie in den beigefügten Ansprüchen behandelt und beschrieben sind.

Die Ausführungsformen, Merkmale und Kombinationen von Merkmalen, wie sie hier in Verbindung mit der Erfindung beschrieben werden, sowie die Kombination von Merkmalen, wie sie in den beigefügten Ansprüchen angegeben sind, aber auch jede Kombination von Merkmalen, wie sie in Verbindung mit den Ausführungsformen erwähnt und beschrieben wird, gelten als hierin offenbart, zumindest jedoch als durch einen Fachmann herleitbar. Insbesondere kann jedes Merkmal und jede Kombination von Merkmalen in den hier beschriebenen Ausführungsformen beispielsweise in einer anderen Kombination, insbesondere in einer anderen Anspruchskategorie, beansprucht werden, mindestens weil die fachkundige Person erkennen wird, dass jede einzelne Kombination der hier genannten Merkmale geeignet ist, zur Lösung des zugrunde liegenden Problems beizutragen.

Des Weiteren können jedes Merkmal und jede Kombination von Merkmalen in den Ansprüchen und in der untenstehenden Beschreibung unabhängig von dem jeweils beanspruchten Gegenstand, unabhängig von Anspruchsabhängigkeiten und Rückverweisen sowie unabhängig von der Anspruchskategorie, in der das Merkmal beansprucht wird, verwendet und separat beansprucht werden. Beispielsweise kann in einer willkürlichen Kombination, die aus einem oder mehreren Ansprüchen ausgewählt ist, eine oder mehrere Ausführungsformen gemäß der untenstehenden Beschreibung und/oder den beigefügten Abbildungen vorgesehen sein.

Die oben beschriebenen Aufgaben werden erfindungsgemäß gelöst durch eine medizinischen Pumpe nach der vorliegenden Erfindung. Eine solche medizinische Pumpe umfasst eine Komponente zur kontaktlosen Datenübertragung von Pumpendaten an ein mobiles Endgerät, wobei die Komponente die Pumpendaten umfasst, und wobei die Pumpendaten in einem für die Übertragung oder Speicherung geeigneten Datenpaket für die Verarbeitung oder Analyse durch nachgelagerte System oder Anwendungen, insbesondere eine Serviceeinrichtung, strukturiert sind.

Mit dem Begriff, dass "die Komponente die Pumpendaten umfasst", soll hierin bevorzugt ausgedrückt werden, dass die Komponente zur kontaktlosen Datenübertragung die Fähigkeit besitzt, relevante Pumpendaten zu speichern und zu übertragen. Diese Pumpendaten können Informationen wie Fehlerdaten, Fehleranalysedaten, Gerätestatusdaten und Gerätetypdaten umfassen. Die Speicherung und Übertragung dieser Daten erfolgt sicher und effizient, um eine zuverlässige Kommunikation mit einem mobilen Endgerät zu gewährleisten. Dabei ist es bevorzugt, dass die Daten wenigstens zeitweise in der Komponente zur kontaktlosen Datenübertragung gespeichert sind. Es ist im Rahmen der vorliegenden Erfindung möglich, dass die Daten ursprünglich an anderer Stelle erzeugt und/oder gespeichert werden, beispielsweise in einer Kontrolleinheit, bevor sie zur kontaktlosen Übertragung an die Komponente weitergeleitet werden.

Gemäß einer bevorzugten Ausführungsform umfassen die Pumpendaten zusätzlich oder alternativ Identifikationsdaten der medizinischen Pumpe, insbesondere eine Seriennummer, ein Herstellungsdatum und/oder einen Pumpentyp, bevorzugt in einer Form, die eine eindeutige Zuordnung des Geräts ermöglicht. Dadurch kann die kontaktlose Datenübertragung nicht nur für Fehlersuche, sondern auch für Service- und Prüfprozesse genutzt werden, bei denen eine schnelle, fehlerarme Identifikation des Geräts erforderlich ist.

In einer weiteren bevorzugten Ausführungsform umfassen die Pumpendaten zusätzlich oder alternativ Statusinformationen zu einem Service- oder Prüfzustand der Pumpe, beispielsweise Informationen darüber, ob eine sicherheitstechnische Kontrolle (STK) oder eine sonstige Prüfung/Wartung bereits durchgeführt wurde und/oder ob eine solche Prüfung fällig ist. Damit kann ein Anwender den Prüfstatus der Pumpe mittels des mobilen Endgeräts kontaktlos auslesen und dokumentieren, ohne dass hierzu Typenschilddaten manuell erfasst oder visuelle Markierungen am Gerät angebracht bzw. ausgewertet werden müssen.

In einer weiteren bevorzugten Ausführungsform ist die Komponente zur kontaktlosen Datenübertragung dazu eingerichtet, neben einem Auslesen von Pumpendaten auch ein kontaktloses Empfangen von Daten von dem mobilen Endgerät zu ermöglichen, insbesondere im Rahmen einer bi-direktionalen kontaktlosen Kommunikation. Dadurch kann ein mittels des mobilen Endgeräts erzeugter oder bereitgestellter Dateneintrag zumindest teilweise auf der Komponente gespeichert werden. Ein solcher Dateneintrag kann insbesondere einen Service- und/oder Prüfstatus der medizinischen Pumpe umfassen, beispielsweise eine Information darüber, dass eine Prüfung, Wartung oder sicherheitstechnische Kontrolle durchgeführt wurde und/oder wann eine solche Maßnahme durchgeführt wurde. Dadurch kann ein aktualisierter Status direkt der medizinischen Pumpe zugeordnet und später kontaktlos ausgelesen werden, wodurch Service-, Wartungs- und/oder Prüfprozesse unterstützt werden können.

Die oben beschriebenen Aufgaben werden ebenfalls erfindungsgemäß gelöst durch eine App nach der vorliegenden Erfindung. Eine solche App ist dazu eingerichtet, bevorzugt auf einem mobilen Endgerät, die Pumpendaten, die von einer Komponente zur kontaktlosen Datenübertragung einer medizinischen Pumpe an das mobile Endgerät übertragen werden, dem Benutzer darzustellen. Gegebenenfalls kann die App auch die Pumpendaten teilweise oder vollständig analysieren oder auswerten. Ebenfalls ist im Rahmen der vorliegenden Erfindung daran gedacht, dass zusätzlich oder alternativ die App dazu eingerichtet ist, die von der Komponente zur kontaktlosen Datenübertragung der medizinischen Pumpe an das mobile Endgerät übertragene Pumpendaten an eine Serviceeinrichtung übertragen zu können. Dies erhöt die Benutzerfreundlichkeit und verringert die Fehleranfälligkeit durch Benutzerfehler erheblich. Die automatische Übertragung der Daten ermöglicht zudem eine zeitnahe Analyse und Bearbeitung der Daten durch die Serviceeinrichtung.

In einer besonders bevorzugten Ausführungsform der vorliegenden Erfindung, ist die App dazu eingerichtet, auf dem mobilen Endgerät die ausgelesenen Daten zu filtern und/oder zu sortieren.

Durch diese Funktion wird die Effizienz und Genauigkeit der Datenanalyse erheblich verbessert. Nutzer können gezielt relevante Informationen aus einer großen Menge an Daten extrahieren, was die Diagnose der medizinischen Pumpe vereinfachen kann. Zudem ermöglicht die Filter- und Sortierfunktion eine schnelle Identifizierung von Fehlern oder Anomalien, wodurch die Reaktionszeit bei Problemen verkürzt wird.

In einer weiteren bevorzugten Ausführungsform der vorliegenden Erfindung, ist die App dazu eingerichtet, eine Benachrichtigung an den Benutzer zu senden, sobald die Daten erfolgreich übertragen wurden.

Diese Funktion erhöht die Benutzerfreundlichkeit und Sicherheit, indem sie den Benutzer sofort über den erfolgreichen Abschluss der Datenübertragung informiert. Dadurch wird gewährleistet, dass der Benutzer über den Status der Datenübertragung informiert ist und bei Bedarf, insbesondere einer unvollständigen Datenübertragung, reagieren kann.

In einer weiteren bevorzugten Ausführungsform der vorliegenden Erfindung, ist die App dazu eingerichtet, einen benutzerdefinierten Kommentar zu den übertragenen Daten hinzuzufügen und zu übertragen. Diese Funktion ermöglicht eine individuelle Anpassung und zusätzliche Kontextualisierung der Daten durch den Benutzer. Der Benutzer kann spezifische Anmerkungen oder Kommentare zu den übertragenen Daten hinzufügen, die für die weitere Analyse oder Dokumentation relevant sind. Dies trägt zur Verbesserung der Kommunikation und Zusammenarbeit zwischen dem Benutzer und der Serviceeinrichtung bei, da zusätzliche Informationen bereitgestellt werden, die die Interpretation und Verarbeitung der Daten erleichtern.

In einer weiteren bevorzugten Ausführungsform der vorliegenden Erfindung, weist die App eine Schnittstelle zur Integration mit anderen Krankenhausinformationssystemen auf. Dadurch können die übertragenen Pumpendaten direkt in die zentralen Informationssysteme integriert und dort weiterverarbeitet werden. Dies führt zu einer verbesserten Datenverfügbarkeit und -konsistenz, da alle relevanten Informationen an einem Ort zusammengeführt werden.

In einer bevorzugten Ausführungsform ist die App dazu eingerichtet, aus den kontaktlos ausgelesenen Pumpendaten Identifikationsdaten (z. B. Seriennummer, Pumpentyp) und/oder Service- bzw. Prüfstatusinformationen (z. B. STK-Status) zu extrahieren und in einem Prüf- oder Serviceprotokoll darzustellen. Die App kann insbesondere dazu eingerichtet sein, diese Daten automatisiert in eine Dokumentation der Medizintechnik zu übernehmen und/oder an ein weiteres IT-System zu übertragen, beispielsweise an einen PC-Arbeitsplatz oder an ein Krankenhausinformationssystem, um den Dokumentationsaufwand zu reduzieren und Eingabefehler zu vermeiden.

Weiter bevorzugt ist die App dazu eingerichtet, einen Hinweis oder eine Kennzeichnung auszugeben, ob eine Pumpe im Rahmen eines Prüfprozesses bereits erfasst wurde und/oder ob ein Prüfstatus vorliegt. Dadurch kann ein Anwender bei der Erfassung einer Mehrzahl von Pumpen eine unmittelbare Rückmeldung erhalten, ohne auf manuelle Markierungen oder nachgelagerte Abgleiche angewiesen zu sein.

Die App kann insbesondere erfindungsgemäß eine Kommunikationsmöglichkeit zwischen Serviceeinrichtung und Nutzer der App umfasse, bspw. kann ein Screen Mirroring und/oder eine Chatoption erlauben, dass Mitarbeiter der Serviceeinrichtung unmittelbar auf die App und/oder die Pumpendaten zugreifen bzw. mit dem Nutzer der App Informationen, bevorzugt in Echtzeit, teilen können.

Die oben beschriebenen Aufgaben werden ebenfalls erfindungsgemäß gelöst durch ein System zur kabellosen Übertragung von Pumpendaten nach der vorliegenden Erfindung. Ein solches erfindungsgemäßes System umfasst eine medizinische Pumpe nach der vorliegenden Erfindung, die eine Komponente zur kontaktlosen Datenübertragung von Pumpendaten an ein mobiles Endgerät umfasst, wobei die Komponente die Pumpendaten umfasst. Das System umfasst weiter ein mobiles Endgerät, das als Reader für die Pumpendaten zur kontaktlosen Datenübertragung der Übertragungskomponente an das mobile Endgerät dient und eine App nach der vorliegenden Erfindung. Das System umfasst weiter eine Serviceeinrichtung, die Pumpendaten von dem mobilen Endgerät empfängt und bevorzugt auch anaylsiert. Es soll dabei verstanden werden, dass sowohl die App als auch die Seviceeinrichtung dazu eingerichtet sein kann gleichzeitig oder unabhängig voneinander Analysen und/oder Teilanalysen durchzuführen.

Die oben beschriebenen Aufgaben werden ebenfalls erfindungsgemäß gelöst durch ein Verfahren zur kabellosen Übertragung von Pumpendaten nach der vorliegenden Erfindung. Ein solches erfindungsgemäßes Verfahren umfasst
- einen Schritt a) des Bereitstellens einer medizinischen Pumpe, die bevorzugt eine medizinische Pumpe nach der vorliegenden Erfindung ist, mit einer Komponente zur kontaktlosen Datenübertragung,
- einen Schritt b) des Übertragens von Pumpendaten der medizinischen Pumpe an ein mobiles Endgerät mittels der Komponente zur kontaktlosen Datenübertragung,
- vorzugsweise eine Schritt c) des Übertragens der in Schritt b) ausgelesenen Pumpendaten von dem mobilen Endgerät (3) an eine Serviceeinrichtung (4).

Der Schritt b) des Übertragens von Pumpendaten der medizinischen Pumpe an ein mobiles Endgerät mittels der Komponente zur kontaktlosen Datenübertragung kann dabei bevorzugt einen Schritt der Prüfung bzw. Validierung der Datenübertragung umfassen. Insbesondere kann dadurch eine fehlerfreie Datenübertragung gewährleistet werden oder auf eine fehlerhafte Datenübertragung hingewiesen werden. Bspw. kann die fehlerfreie Übertragung von Daten oder Datenpaketen mittels einer Prüfsumme validiert werden.

Es ist dabei bevorzugt, dass die Pumpendaten in einem für die Übertragung oder Speicherung geeigneten Datenpaket strukturiert sind, insbesondere für die Verarbeitung und Analyse der Daten durch nachgelagerte Systeme oder Anwendungen, insbesondere eine Serviceeinrichtung. Durch die Strukturierung der Daten in geeigneten Paketen kann die Datenintegrität verbessert werden, da das Risiko von Datenverlust oder -beschädigung während der Übertragung minimiert wird. Zudem erhöht sich die Übertragungsgeschwindigkeit, da die Daten in einem standardisierten Format vorliegen, was eine schnellere und effizientere Übertragung ermöglicht. Strukturierte Datenpakete erleichtern auch die Verarbeitung und Analyse der Daten durch nachgelagerte Systeme oder Anwendungen. Bei Problemen können strukturierte Datenpakete leichter überprüft und analysiert werden, um Fehlerquellen zu identifizieren. Insbesondere erlaubt die Bereitstellung und Übertragung von Datenpaketen, dass im Rahmen der Erfindung auch Komponenten zur Datenübertragung genutzt werden können, die ggf. für die Übertragung größerer Datenmengen nicht geeignet sind.

Es ist dabei weiter bevorzugt, dass das Datenpaket einen zeitlichen Ausschnitt um ein Ereignis und/oder bestimmte Messwerte umfasst, die sich auf Komponenten wie den Antrieb oder die Elektronik beziehen, ausgewählt ist.

Dies ermöglicht eine gezielte und präzise Analyse der relevanten Daten, da spezifische Ereignisse oder Messwerte isoliert und untersucht werden können. Durch die Fokussierung auf bestimmte Zeiträume oder Messwerte wird die Fehlersuche erleichtert und die Diagnosegenauigkeit erhöht. Zudem können durch die gezielte Auswahl von Datensegmenten Ressourcen effizienter genutzt werden, da nur relevante Daten übertragen und gespeichert werden.

In einer bevorzugten Ausführungsform umfasst das Verfahren zusätzlich einen Schritt des Auslesens von Protokoll- bzw. Logdaten der medizinischen Pumpe (insbesondere einer Log-Datei) durch das mobile Endgerät mittels der kontaktlosen Datenübertragungskomponente, wobei das Auslesen bevorzugt vor Ort am Einsatzort der Pumpe erfolgt. Die ausgelesenen Logdaten können anschließend in der App auf dem mobilen Endgerät dargestellt und/oder einer Erstanalyse unterzogen werden, um eine erste Einordnung eines Fehlers oder Ereignisses zu unterstützen.

Zusätzlich oder alternativ kann die App die ausgelesenen Logdaten in einem Analysemodus aufbereiten, insbesondere durch Filterung, Sortierung und/oder Erstellung eines Berichts aus den ausgelesenen Daten. Dadurch kann eine erste technische Beurteilung durch den Anwender ermöglicht werden, ohne dass hierfür zwingend ein Transport der Pumpe oder ein Zugriff auf eine stationäre Auswerteumgebung erforderlich ist, wobei eine weitergehende Analyse weiterhin durch eine Serviceeinrichtung erfolgen kann.

Bevorzugt ist die medizinische Pumpe und/oder die Komponente dazu eingerichtet, dass die Pumpendaten stromlos, insbesondere bei einem Ausfall der Stromversorgung, ausgelesen werden können.

Mit dem Begriff "kontaktlos", wie hierin verwendet, ist bevorzugt eine Übertragung von Daten oder Energie ohne physische Verbindung zwischen den beteiligten Geräten gemeint. In diesem Kontext bedeutet "kontaktlos", weiter bevorzugt "direkt kontaktlos", so dass die Übertragung unmittelbar und ohne jegliche physische Verbindung zwischen der Komponente und dem mobilen Endgerät erfolgt. Dies schließt mittelbare kontaktlose Übertragungen aus, bei denen Daten oder Energie über Zwischenstationen oder zusätzliche Geräte übertragen werden. Ein Beispiel für eine direkte kontaktlose Übertragung ist die Nutzung von NFC- oder RFID-Technologie, bei der die Daten direkt zwischen der Pumpe und dem mobilen Endgerät ausgetauscht werden.

Der Begriff "medizinische Pumpe", wie hierin verwendet, meint bevorzugt ein Gerät, das zur kontrollierten Verabreichung von Flüssigkeiten, wie Medikamenten oder Nährlösungen, an Patienten eingesetzt wird. Es gibt verschiedene Arten von medizinischen Pumpen, die jeweils für spezifische Anwendungen und Anforderungen entwickelt wurden. Zu den gängigen medizinischen Pumpen gehören beispielsweise Infusionspumpen, die zur kontinuierlichen oder intermittierenden Verabreichung von Flüssigkeiten über einen längeren Zeitraum verwendet werden; Insulinpumpen die speziell für die kontinuierliche Abgabe von Insulin bei Diabetespatienten konzipiert sind; Schmerzpumpen, die auch als PCA-Pumpen (Patient Controlled Analgesia) bekannt sind und es Patienten ermöglichen, selbstständig Schmerzmittel zu verabreichen; Ernährungspumpen, die zur Verabreichung von Nährlösungen bei Patienten eingesetzt werden, die nicht in der Lage sind, ausreichend Nahrung oral aufzunehmen; Spritzenpumpen, die eine präzise Dosierung von Medikamenten ermöglichen; und Herzpumpen, die bei der Unterstützung der Herzfunktion eingesetzt werden.

Medizinische Pumpen sind oft mit verschiedenen Sensoren und Überwachungssystemen ausgestattet, um ihre Funktionalität und Sicherheit zu gewährleisten. Dabei generieren die Pumpen verschiedene Daten, die für die Analyse der Pumpe, insbesondere von Gerätefunktionalitäten, Performance oder Fehlern notwendig sein können. Deratige Pumpendaten umfassen gemäß der vorliegenden Erfindung unter anderen Fehlerdaten, Fehleranalysedaten, Gerätestatusdaten, Gerätetypdaten, Leistungsdaten, Zeitstempel, Eingabeprotokolle, Alarme und Informationen zu einem verwendeten Einmalartikel. Ein Zeitstempel kann dabei insbesondere vorteilhaft erlauben, den Zeitpunkt eines Ereignisses oder eines anderen Pumpendateneintrags zeitlich festzustellen und/oder einzuordnen. Mit anderen Worten kann mittels des Zeitstempels festgestellt werden, wann genau was geschehen ist. Mit einem Eingabeprotokoll, wie hierin verwendet, soll bevorzugt gemeint sein, dass die Eingabe durch einen Benutzer protokolliert ist. Bspw. ist in dem Eingabeprotokoll erfasst, welche Tastendrücke ein Nutzer vorgenommen hat. Alarme können sowohl zeitlich, als auch nach Typen in den Pumpendaten erfasst sein, insbesondere können Alarme in verschiedene Alarmetypen, bpw. als Operational Alarm, Device Alarm, Pre Alarm, Caution Alarm, erfasst sein.

Durch die vorliegende Erfindung wird eine medizinische Pumpe bereitgestellt, die eine Komponente zur kontaktlosen Datenübertragung derartiger Pumpendaten an ein mobiles Endgerät umfasst. Dies bietet zahlreiche Vorteile. Beispielsweise kann durch die Echtzeit-Datenübertragung Pumpendaten sofort an das mobile Endgerät gesendet werden, was eine unmittelbare Überwachung und Analyse ermöglicht. Dies ist besonders wichtig, um schnell auf eventuelle Fehlfunktionen oder Abweichungen reagieren zu können. Die kontaktlose Datenübertragung erhöht zudem die Effizienz und Anwendbarkeit durch das Klinikpersonal, da das manuelle Auslesen und Übertragen der Daten entfällt. Die Pumpe kann insbesondere vor Ort bleiben, während die Pumpendaten, bspw. zur Fehleranalyse, ausgelesen und analysiert werden. Dies spart Zeit und reduziert die Arbeitsbelastung des Klinikpersonals erheblich.

Ein weiterer Vorteil ist die erhöhte Mobilität und Flexibilität. Da die Daten an ein mobiles Endgerät übertragen werden, können sie von überall aus eingesehen und analysiert werden. Durch die sofortige Verfügbarkeit der Pumpendaten können Fehler schneller identifiziert und behoben werden, was die Ausfallzeiten der Pumpe reduziert und die Kontinuität der Patientenversorgung verbessert. Insbesondere erlaubt die vorliegende Erfindung, dass die Daten auf besonders einfache Weise, und damit auch von ungeschultem Personal, ausgelesen werden können. Darüber hinaus ist ein Auslesen von Daten nicht davon abhängig, ob eine Verbindung mit einem Netwerk, insbesondere dem Internet, besteht oder nicht. Die Daten können manuell ausgelesen werden, auch wenn die medizinische Pumpe nicht online ist.

Es sei im Zusammenhang mit dem erfindungsgemäßen Verfahren auch darauf hingewiesen, dass die angegebenen Schritte nicht zwangsläufig in der angegebenen Reihenfolge durchgeführt werden müssen. Die angegebenen Schritte können in jeder anderen geeigneten Reihenfolge oder auch gleichzeitig durchgeführt werden.

Allerdings kann die oben angegebene Reihenfolge für bestimmte Ausführungsformen des erfindungsgemäßen Verfahrens vorteilhaft sein.

Es wird zudem von einem Fachmann anerkannt werden, dass ein Merkmal, eine Ausführungsform, eine Wirkung oder ein Vorteil, wie hier in Verbindung mit der erfinderischen Vorrichtung und/oder der erfinderischen App und/oder dem erfinderischen System beschrieben, auch ein Merkmal, eine Ausführungsform, eine Wirkung oder ein Vorteil des erfinderischen Verfahrens sein kann, bzw. umgekehrt.

Der Begriff "Komponente zur kontaktlosen Datenübertragung", wie hierin verwendet, bezeichnet bevorzugt ein Bauteil oder eine Einheit, die in der Lage ist, Daten ohne physische Verbindung zu übertragen. Diese Komponente kann verschiedene Technologien umfassen, wie beispielsweise NFC (Near Field Communication) oder RFID (Radio Frequency Identification), die es ermöglichen, Daten drahtlos an ein mobiles Endgerät zu senden und zu empfangen.

Solche Komponenten sind besonders nützlich in medizinischen Pumpen, da sie eine schnelle und effiziente Übertragung von Pumpendaten ermöglichen. Dies reduziert die Notwendigkeit manueller Eingriffe und minimiert das Risiko von Übertragungsfehlern. Darüber hinaus können diese Komponenten mit Sicherheitsmerkmalen ausgestattet werden, um unautorisierten Zugriff auf die Daten zu verhindern, was die Datensicherheit erhöht. Die kontaktlose Datenübertragung bietet somit eine verbesserte Benutzerfreundlichkeit und erhöht die Effizienz und Sicherheit im medizinischen Umfeld.

Der Begriff "mobiles Endgerät", wie hierin verwendet, bezeichnet bevorzugt ein tragbares elektronisches Gerät, das zur drahtlosen Kommunikation und Datenverarbeitung fähig ist. Solche Geräte umfassen typischerweise Smartphones, Tablets, Laptops und ähnliche tragbare Computer, die mit entsprechenden Schnittstellen ausgestattet sind, um Daten von anderen Geräten, wie beispielsweise medizinischen Pumpen, zu empfangen und zu verarbeiten.

Diese mobilen Endgeräte sind in der Lage, Daten über verschiedene drahtlose Technologien wie NFC, RFID, Bluetooth oder WLAN zu empfangen. Sie bieten eine benutzerfreundliche Oberfläche zur Anzeige und Analyse der empfangenen Daten und ermöglichen es dem Klinikpersonal, schnell und effizient auf wichtige Informationen zuzugreifen. Durch die Verwendung mobiler Endgeräte wird die Flexibilität und Mobilität des Klinikpersonals erhöht, da sie die Daten von überall aus einsehen und verwalten können.

In einer bevorzugten Ausführungsform der vorliegenden Erfindung umfasst die Komponente zur kontaktlosen Datenübertragung einen NFC-Chip und/oder einen RFID-Chip, der eingerichtet ist zum Auslesen der Pumpendaten mittels des mobilen Endgeräts.

Dies ist insbesondere vorteilhaft, da die Verwendung eines NFC-Chips und/oder eines RFID-Chips in der Komponente zur kontaktlosen Datenübertragung eine schnelle und zuverlässige Übertragung der Pumpendaten ermöglicht. Diese Technologien sind bekannt für ihre hohe Übertragungsgeschwindigkeit und Zuverlässigkeit, was bedeutet, dass die Daten nahezu in Echtzeit an das mobile Endgerät übermittelt werden können. Dadurch kann das Klinikpersonal sofort auf wichtige Informationen zugreifen und entsprechend reagieren bzw. diese unmittelbar an die Serviceeinrichtung übermitteln.

Im Rahmen der vorliegenden Erfindung umfasst die medizinische Pumpe bevorzugt einen Mikroprozessor, Mikrokontroller und/oder eine andere Recheneinheit, zur Erzeugung der Pumpendaten und/oder ggf. zur Übertragung der Pumpendaten auf die Komponente.

In einer Ausführungsform der vorliegenden Erfindung ist die medizinische Pumpe dazu eingerichtet, Pumpendaten zu erfassen und auf der Komponente zu speichern. Dabei kann das Erfassen und/oder das Speichern der Daten auf der Komponente regelmäßig, das heisst bspw. in regelmäßigen Zeitabschnitten oder bei Erzeugung der Daten, erfolgen.

In einer weiteren bevorzugten Ausführungsform der vorliegenden Erfindung ist die medizinische Pumpe dazu eingerichtet, Pumpendaten nutzerbasiert und/oder eventbasiert zu erfassen.

Dies ist insbesondere vorteilhaft, da die medizinische Pumpe durch die nutzerbasierte und/oder eventbasierte Erfassung von Pumpendaten eine präzisere und individuellere Überwachung und Steuerung ermöglicht. Nutzerbasierte Datenerfassung bedeutet dabei im Rahmen der vorliegenden Erfindung, wie hierin verwendet bevorzugt, dass die Pumpe Daten sammelt, die durch den Nutzer verursacht werden. Dies kann beispielsweise die Anpassung der Pumpenleistung an die spezifischen Bedürfnisse und das Verhalten des jeweiligen Nutzers umfassen, was zu einer optimierten und personalisierten Behandlung führt.

Eventbasierte Datenerfassung hingegen bedeutet dabei im Rahmen der vorliegenden Erfindung, wie hierin verwendet bevorzugt, dass die Pumpe auf bestimmte Ereignisse oder Zustände, insbesondere Fehler, reagiert und entsprechende Daten erfasst. Dies kann beispielsweise der Fall sein, wenn ein Fehler auftritt oder sich der Betriebszustand der Pumpe ändert. Durch die Erfassung solcher Ereignisse können potenzielle Probleme frühzeitig erkannt und behoben werden, bevor sie zu größeren Störungen führen. Die Kombination beider Ansätze bietet eine umfassende und flexible Lösung zur Datenerfassung, die sowohl die Effizienz als auch die Sicherheit der Pumpe erhöht. Ein Anwender, insbesondere Klinikpersonal, Rettungsdienste oder andere, kann auf detaillierte und relevante Daten zugreifen, die ihnen helfen, fundierte Entscheidungen zu treffen und die Patientenversorgung zu verbessern.

In einer weiteren bevorzugten Ausführungsform der vorliegenden Erfindung ist die Pumpe dazu eingerichtet, Pumpendaten nutzerbasiert und/oder eventbasiert auf der Komponente zur kontaktlosen Datenübertragung zu speichern. Dies bedeutet, dass die Pumpe in der Lage ist, Daten basierend auf den Aktionen des Nutzers oder bestimmten Ereignissen zu erfassen und zu speichern. Nutzerbasierte Daten können beispielsweise durch Eingaben oder Interaktionen des Nutzers generiert werden, während eventbasierte Daten durch spezifische Ereignisse oder Zustände der Pumpe erfasst werden. Diese Daten werden dann auf der Komponente zur kontaktlosen Datenübertragung gespeichert, um eine effiziente und sichere Übertragung an ein mobiles Endgerät zu ermöglichen. Mit anderen Worten ist im Rahmen der vorliegenden Erfindung daran gedacht, dass ein Nutzer - nutzerbasiert - das Speichern der Daten auf der Komponente zur kontaktlosen Datenübertragung gezielt auslösen kann. Gleichzeitig oder alternativ können bestimmte, ggf. vorbestimmte Ergeinisse - eventbasiert -, bspw. ein Alarm, eine Unterbrechung der Stromversorgung oder ähnliches, ein Speichern der Daten auf der Komponente zur kontaktlosen Datenübertragung auslösen.

Dies ist insbesondere vorteilhaft, da die Speicherung von Pumpendaten auf der Komponente zur kontaktlosen Datenübertragung sowohl nutzerbasiert als auch eventbasiert eine effiziente und flexible Datenverwaltung ermöglicht. So kann insbesondere eine Log-Datei erstellt werden, die Pumpendaten umfasst.

Die Speicherung der Daten direkt auf der Komponente zur kontaktlosen Datenübertragung bietet dabei den zusätzlichen Vorteil, dass die Daten jederzeit und überall verfügbar sind, ohne dass eine Internetverbindung erforderlich ist. Dies erhöht die Flexibilität und Mobilität des Klinikpersonals, da sie die Daten einfach mit einem mobilen Endgerät auslesen und analysieren können.

In einer weiteren bevorzugten Ausführungsform der vorliegenden Erfindung ist dabei die Datenübertragung von Pumpendaten von der Komponente zur kontaktlosen Datenübertragung an ein mobiles Endgerät von einer Internetverbindung der Pumpe unabhängig.

In einer weiteren bevorzugten Ausführungsform der vorliegenden Erfindung ist die Komponente zur kontaktlosen Datenübertragung durch wenigstens ein Sicherheitsmerkmal vor einem unauthorisierten Auslesen der Pumpendaten mittels des mobilen Endgeräts geschützt.

Der Begriff "Sicherheitsmerkmal", wie hierin verwendet, bezeichnet bevorzugt eine Maßnahme oder ein Verfahren, das dazu dient, den unautorisierten Zugriff auf Daten oder Systeme zu verhindern. Solche Sicherheitsmerkmale können verschiedene Formen annehmen, einschließlich, aber nicht beschränkt auf, Passwörter, gezielte Autorisierungen und Freischaltcodes. Ein Passwort kann erforderlich sein, um den Zugriff auf die Daten zu ermöglichen, während gezielte Autorisierungen sicherstellen, dass nur berechtigte Nutzer Zugriff auf die Pumpendaten haben. Ein Freischaltcode kann als zusätzliche Sicherheitsstufe dienen, die vor dem Auslesen der Daten eingegeben werden muss. Diese Sicherheitsmerkmale tragen dazu bei, die Integrität und Vertraulichkeit der Pumpendaten zu gewährleisten, indem sie unautorisierten Zugriff verhindern und sicherstellen, dass nur autorisierte Personen die Daten einsehen und nutzen können.

Ein Sicherheitsmerkmal kann dabei bevorzugt an der medizinischen Pumpe gemäß der vorliegenden Erfindung und/oder in der App gemäß der vorliegenden Erfindung vorgesehen sein.

Der Begriff "Störung", wie hierin verwendet, bezeichnet bevorzugt jede Art von unerwünschter Beeinflussung oder Unterbrechung, die die Funktionalität oder Leistung eines Geräts oder Systems negativ beeinflusst. Dies kann elektromagnetische Interferenzen, mechanische Vibrationen, Temperaturänderungen oder andere äußere Einflüsse umfassen, die die ordnungsgemäße Funktion der Komponente zur kontaktlosen Datenübertragung beeinträchtigen könnten.

In einer weiteren bevorzugten Ausführungsform der vorliegenden Erfindung ist die Komponente zur kontaktlosen Datenübertragung in einem vor Störungen abgeschirmten Bereich der medizinischen Pumpe angeordnet.

Dies bedeutet, dass die Komponente durch spezielle Maßnahmen oder Materialien geschützt ist, die verhindern, dass äußere Störungen die Datenübertragung beeinträchtigen.

Diese Abschirmung ist besonders vorteilhaft, da sie die Zuverlässigkeit und Genauigkeit der Datenübertragung erhöht. Durch den Schutz vor Störungen wird sichergestellt, dass die Pumpendaten korrekt und ohne Unterbrechungen an das mobile Endgerät übertragen werden können. Dies trägt zur Gesamtzuverlässigkeit der medizinischen Pumpe bei und stellt sicher, dass das Klinikpersonal jederzeit auf präzise und verlässliche Daten zugreifen kann.

In einer weiteren bevorzugten Ausführungsform der vorliegenden Erfindung werden in einem Schritt c) die in Schritt b) ausgelesenen Pumpendaten von dem mobilen Endgerät an eine Serviceeinrichtung übertragen. Der Begriff "Serviceeinrichtung", wie hierin verwendet, bezeichnet bevorzugt eine Einrichtung oder ein System, das zur Verarbeitung, Analyse und Überwachung von Daten dient. Eine Serviceeinrichtung kann ein Server, ein Computer, ein zentrales Datenmanagementsystem, eine Cloud-Plattform oder ähnliches, aber auch ein anderes medizinisches Gerät sein, das die Daten für weitere Analysen oder zur Überwachung verwendet. Diese Einrichtung dient als zentraler Ort, an dem die Daten gesammelt, gespeichert und ausgewertet werden können. Durch die Übertragung der Daten an eine Serviceeinrichtung wird eine umfassende und effiziente Datenverarbeitung ermöglicht, die zur Verbesserung der Patientenversorgung und zur Optimierung der Pumpenleistung beiträgt.

In einer weiteren bevorzugten Ausführungsform der vorliegenden Erfindung erfolgt die Übertragung der Pumpendaten an die Serviceeinrichtung in einem Schritt c) verschlüsselt. Der Begriff "verschlüsselt", wie hierin verwendet, bezeichnet bevorzugt eine Methode der Datenübertragung, bei der die Daten in eine codierte Form umgewandelt werden, um sie vor unautorisiertem Zugriff zu schützen. Diese Verschlüsselung stellt sicher, dass nur autorisierte Empfänger, die über den entsprechenden Entschlüsselungsschlüssel verfügen, die Daten lesen und verarbeiten können.

In einer weiteren bevorzugten Ausführungsform der vorliegenden Erfindung werden die in einem Schritt b) ausgelesenen Pumpendaten in einer App auf dem mobilen Endgerät dargestellt. Dies ist insbesondere vorteilhaft, da die Darstellung der in einem Schritt b) ausgelesenen Pumpendaten in einer App auf dem mobilen Endgerät eine sofortige und benutzerfreundliche Visualisierung der Daten ermöglicht. Durch die Nutzung einer App können die Daten in Echtzeit angezeigt werden, was eine schnelle und effiziente Überwachung der Pumpenleistung und des Gerätezustands erlaubt. Die App bietet eine intuitive Benutzeroberfläche, die es dem Klinikpersonal erleichtert, die relevanten Informationen schnell zu erfassen und zu interpretieren. Dies reduziert die Notwendigkeit für umfangreiche Schulungen und ermöglicht es dem Personal, sich auf die Patientenversorgung zu konzentrieren. Darüber hinaus können durch die App benutzerdefinierte Alarme und Benachrichtigungen eingerichtet werden, die das Personal sofort auf kritische Zustände oder Fehler der medizinischen Pumpe aufmerksam machen.

Ein weiterer Vorteil ist die Mobilität und Flexibilität, die durch die Nutzung eines mobilen Endgeräts und einer App erreicht wird. Das Klinikpersonal kann die Pumpendaten von überall aus einsehen und verwalten, was die Effizienz und Reaktionsfähigkeit erhöht. Zudem können die Daten in der App gespeichert und für spätere Analysen oder Berichte genutzt werden, was die langfristige Überwachung und Optimierung der Pumpenleistung unterstützt.

In einer weiteren bevorzugten Ausführungsform der vorliegenden Erfindung werden die in einem Schritt c) übertragenen Pumpendaten, bevorzugt in der Serviceeinrichtung, ausgewertet.

Der Begriff "auswerten", wie hierin verwendet, bezeichnet bevorzugt den Prozess der Analyse und Interpretation von Daten, um daraus relevante Informationen und Erkenntnisse zu gewinnen. Dies bedeutet, dass die Pumpendaten, nachdem sie an die Serviceeinrichtung übertragen wurden, systematisch analysiert werden, um beispielsweise Fehler zu identifizieren, die Leistung der Pumpe zu überwachen oder Trends und Muster zu erkennen. Die Auswertung kann verschiedene Methoden und Techniken umfassen, wie statistische Analysen, maschinelles Lernen oder andere datenanalytische Verfahren, um die Daten umfassend zu interpretieren und fundierte Entscheidungen zu treffen.

Die Auswertung der Daten in der Serviceeinrichtung ermöglicht es, detaillierte Einblicke in die Funktionsweise und den Zustand der Pumpe zu gewinnen oder Fehler zu erkennen, was zur Verbesserung der Zuverlässigkeit und Effizienz der Pumpe beiträgt oder Hinweise zur Behebung des Fehlers liefern kann.

Im Folgenden werden weitere Ausführungsformen der vorliegenden Erfindung beschrieben, wobei diese Ausführungsformen einzeln oder in beliebiger Kombination miteinander und/oder mit den zuvor beschriebenen Merkmalen vorgesehen sein können:
In einer Ausführungsform umfassen die Pumpendaten Identifikationsdaten der medizinischen Pumpe, insbesondere eine Seriennummer und/oder einen Pumpentyp und/oder ein Herstellungsdatum, wobei diese Identifikationsdaten dazu eingerichtet sind, mittels des mobilen Endgeräts kontaktlos ausgelesen zu werden, um eine eindeutige Identifikation der medizinischen Pumpe zu ermöglichen.

In einer Ausführungsform umfassen die Pumpendaten einen Service- und/oder Prüfstatus der medizinischen Pumpe, insbesondere einen Status einer sicherheitstechnischen Kontrolle und/oder einer Wartung, wobei der Service- und/oder Prüfstatus mittels des mobilen Endgeräts kontaktlos auslesbar ist, um eine Feststellung zu ermöglichen, ob eine Prüfung durchgeführt wurde, durchgeführt worden ist und/oder fällig ist.

In einer Ausführungsform ist die App dazu eingerichtet, kontaktlos ausgelesene Identifikationsdaten und/oder Service- und/oder Prüfstatusdaten der medizinischen Pumpe zu erfassen, darzustellen und/oder in einer Dokumentation zu speichern und/oder an eine Serviceeinrichtung und/oder ein externes Datenverarbeitungssystem zu übertragen, insbesondere zur Unterstützung eines Service-, Wartungs- und/oder Prüfprozesses.

In einer Ausführungsform ist das mobile Endgerät dazu eingerichtet, mittels der Komponente zur kontaktlosen Datenübertragung Logdaten der medizinischen Pumpe kontaktlos auszulesen und diese Logdaten in der App darzustellen und/oder zumindest teilweise zu analysieren, insbesondere um eine Erstanalyse eines Betriebszustands und/oder eines Fehlers der medizinischen Pumpe zu ermöglichen.

In einer Ausführungsform umfasst das Verfahren einen Schritt des kontaktlosen Auslesens von Identifikationsdaten einer Mehrzahl von medizinischen Pumpen mittels des mobilen Endgeräts, wobei die ausgelesenen Identifikationsdaten automatisch gespeichert und/oder an eine Serviceeinrichtung und/oder ein externes Datenverarbeitungssystem übertragen werden, um eine Dokumentation und/oder einen Service-, Wartungs- und/oder Prüfprozess zu unterstützen.

In einer Ausführungsform ist die Komponente zur kontaktlosen Datenübertragung dazu eingerichtet, Pumpendaten kontaktlos zu übertragen, während sich die medizinische Pumpe in ihrem bestimmungsgemäßen Einsatzort befindet, insbesondere ohne dass die medizinische Pumpe ausgebaut oder physisch mit einem externen Auslesegerät verbunden werden muss.

In einer Ausführungsform ist die Komponente zur kontaktlosen Datenübertragung dazu eingerichtet, neben einem kontaktlosen Übertragen von Pumpendaten an das mobile Endgerät auch Daten kontaktlos von dem mobilen Endgerät zu empfangen und zumindest teilweise auf der Komponente zu speichern, insbesondere im Rahmen einer bi-direktionalen kontaktlosen Kommunikation.

In einer Ausführungsform ist das mobile Endgerät und/oder die App dazu eingerichtet, einen Service- und/oder Prüfstatus der medizinischen Pumpe, insbesondere eine Information über eine durchgeführte oder anstehende sicherheitstechnische Kontrolle und/oder Wartung, kontaktlos an die Komponente zur kontaktlosen Datenübertragung zu übertragen, sodass der Service- und/oder Prüfstatus auf der Komponente gespeichert und zu einem späteren Zeitpunkt kontaktlos ausgelesen werden kann.

### Kurzbeschreibung der Figuren

Die vorliegende Erfindung wird im Folgenden ausführlicher erläutert unter Bezugnahme auf die Zeichnungen, aus denen weitere Merkmale, Ausführungsformen und Vorteile entnommen werden können. In den in den Abbildungen gezeigten Ausführungsformen werden Elemente, die ähnliche oder identische Funktionen haben, mit gleichen Bezugszeichen versehen. Es wird darauf hingewiesen, dass die Abbildungen möglicherweise nicht maßstabsgetreu zueinander sind.

Dabei zeigt:

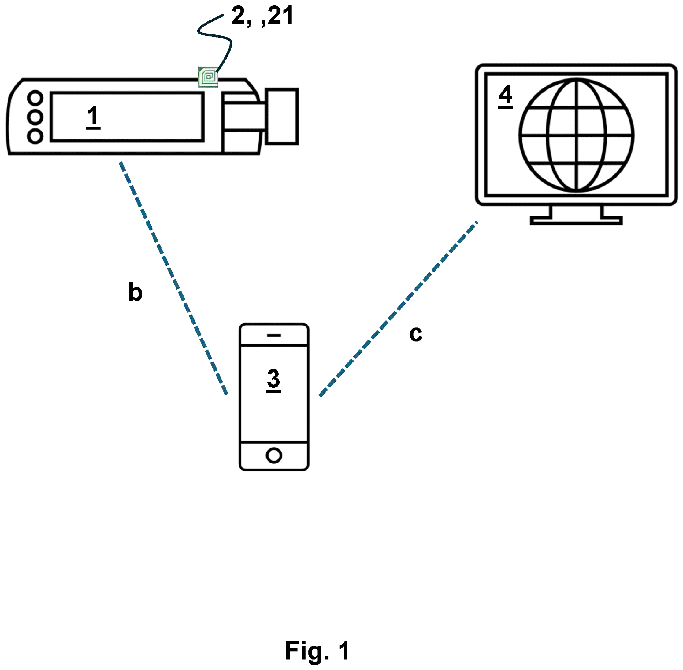

FIG 1 eine schematische Darstellung eines Systems gemäß einer ersten erfindungsgemäßen Ausführungsform.

### Detaillierte Beschreibung

FIG 1 zeigt eine schematische Darstellung eines Systems zur kabellosen Übertragung von Pumpendaten gemäß einer ersten erfindungsgemäßen Ausführungsform der vorliegenden Erfindung. Das System umfasst eine medizinische Pumpe 1 nach der vorliegenden Erfinung, die eine Komponente 2 zur kontaktlosen Datenübertragung von Pumpendaten an ein mobiles Endgerät 3 umfasst. Diese Komponente 2 zur kontaktlosen Datenübertragung kann bspw. ein NFC-Chip 21 und/oder ein RFID-Chip 21 sein, oder einen NFC-Chip 21 und/oder einen RFID-Chip 21 umfassen. Der Chip 21 ist eingerichtet zum Auslesen der Pumpendaten mittels des mobilen Endgeräts 3, hier ein Smartphone 3. Im Falle eines NFC Chips 21, kann die bi-direktionale Kommunikation zwischen Chip 21 und Endgerät 3 dazu genutzt werden, dass sowohl Daten gesendet als auch empfangen werden. Ist der Chip 21 ein RFID Chip, so ist dieser in der Regel unidirektional, wobei die Daten nur vom RFID-Chip 21 zum RFID-Reader, hier das oder im Smartphone 3, fließen können. Der Chip 21 kann vorteilhaft in einem vor Störungen abgeschirmten Bereich der medizinischen Pumpe 1 angeordnet sein.

Die Komponente 2, bzw. der Chip 21, umfasst die Pumpendaten der Pumpe 1. Die Pumpe 1 kann dabei die Pumpendaten insbesondere nutzerbasiert und/oder eventbasiert erfassen.

Das System umfasst weiter das mobile Endgerät 3, das als Reader für die Pumpendaten zur kontaktlosen Datenübertragung des Chips 2 an das mobile Endgerät 3 dient. Auf dem Endgerät 3, bspw. ein Smartphone, ist eine App nach der vorliegenden Erfindung installiert. Via dieser App kann die Serviceeinrichtung 4, die Pumpendaten von dem Smartphone 3 empfangen und dann analysieren. Dabei können unterschiedliche Pumpendaten verwendet und analysiert werden, insbesondere Fehlerdaten, Fehleranalysedaten, Gerätestatusdaten oder auch Gerätetypdaten der Pumpe 1.

Die App auf dem Smartphone 3 ist dabei dazu eingerichtet die Pumpendaten darzustellen, wahlweise zusätzlich oder alternativ auch zu analysieren, die von dem Chip 2 der medizinischen Pumpe 1 an das Smartphone 3 übertragen wurden.

Auch kann die App die von dem Chip 2 der medizinischen Pumpe 1 an das Smartphone 3 übertragene Pumpendaten an die Serviceeinrichtung 4 übertragen. Hierzu wird bevorzugt eine Internetverbindung zwischen der Serviceeinrichtung 4 und dem mobilen Endgeräte 3, also hier des Smartphones, verwendet.

In einer Ausführungsform kann das in FIG 1 dargestellte System ferner zur Unterstützung von Service- und Prüfprozessen, insbesondere wiederkehrenden sicherheitstechnischen Kontrollen (STK), eingesetzt werden. Hierbei werden mittels des mobilen Endgeräts 3 über die Komponente 2 nicht nur Fehler- oder Zustandsdaten, sondern bevorzugt auch Identifikationsdaten der Pumpe 1 (z. B. Seriennummer, Pumpentyp) und/oder ein Service- bzw. Prüfstatus ausgelesen. Dadurch kann ein Anwender eine Mehrzahl von Pumpen effizient erfassen und die erfassten Daten in der App dokumentieren und/oder an die Serviceeinrichtung 4 bzw. ein weiteres IT-System übertragen.

Die App kann dabei bevorzugt eine Rückmeldung ausgeben, ob für die jeweilige Pumpe 1 bereits ein Prüfstatus vorliegt bzw. ob die Pumpe im Rahmen des Prozesses bereits erfasst wurde. Damit kann eine unmittelbare Plausibilisierung der Erfassung erfolgen, ohne dass Typenschildinformationen manuell übertragen oder zusätzliche visuelle Markierungen am Gerät erforderlich sind.

Nach dem erfindungsgemäßen Verfahren wird dabei zunächst die in einem Schritt a) bereitgestellte medizinische Pumpe 1 die Pumpendaten auf den Chip 2 umfassen und dann in einem Schritt b), der in der Fig. 1 als schematische gestrichelte Linie b dargestellt ist, die Pumpendaten der medizinischen Pumpe 1 an das Smartphone 3 mittels des Chips 2 übertragen.

Nachfolgend werden in einem Schritt c die in Schritt b ausgelesenen Pumpendaten von dem Smartphone 3 an die Serviceeinrichtung 4 übertragen.

Es soll verstanden werden, dass in Schritt b die Datenübertragung von Pumpendaten vom Chip 2 an das Smartphone 3 von einer bestehenden Internetverbindung der Pumpe 1 unabhängig ist. Natürlich ist zur Übertragung der Pumpendaten vom Smartphone 3 an die Serviceeinrichtung 4 eine bestehende Datenverbindung, insbesondere Internetverbindung, jedoch vorteilhaft.

Die Pumpe 1 ist in diesem Beispiel dazu eingerichtet, Pumpendaten nutzerbasiert und/oder eventbasiert auf dem Chip 2 zu speichern. Der Chip 2 ist dabei durch wenigstens ein Sicherheitsmerkmal, bspw einen Sicherheitscode, vor einem unauthorisierten Auslesen der Pumpendaten durch das Smartphone 3 geschützt.

In Schritt c), der in der Fig. 1 als schematische gestrichelte Linie c dargestellt ist, erfolgt die Übertragung der Pumpendaten an die Serviceeinrichtung 4. Diese Datenübertragung erfolgt bevorzugt verschlüsselt. Sodann erfolgt die Auswertung der in einem Schritt c übertragenen Pumpendaten in der Serviceeinrichtung 4.

### Referenzzeichenliste

1- Medizinische Pumpe
2- Komponente zur kontaktlosen Datenübertragung
21-NFC-Chip/RFID-Chip
3- Mobiles Endgerät
4- Serviceeinrichtung

## Patentansprüche

1. Medizinische Pumpe (1) umfassend eine Komponente (2) zur kontaktlosen Datenübertragung von Pumpendaten an ein mobiles Endgerät (3), wobei die Komponente (2) die Pumpendaten umfasst, und
wobei die Pumpendaten in einem für die Übertragung oder Speicherung geeigneten Datenpaket für die Verarbeitung oder Analyse durch nachgelagerte Systeme oder Anwendungen, insbesondere eine Serviceeinrichtung (4), strukturiert sind.

2. Medizinische Pumpe (1) nach Anspruch 1, wobei die Komponente (2) zur kontaktlosen Datenübertragung einen NFC-Chip (21) und/oder einen RFID-Chip (21) umfasst, der eingerichtet ist zum Auslesen der Pumpendaten mittels des mobilen Endgeräts (3).

3. Medizinische Pumpe (1) nach Anspruch 1 oder 2, wobei die Pumpe (1) dazu eingerichtet ist, Pumpendaten nutzerbasiert und/oder eventbasiert zu erfassen.

4. Medizinische Pumpe (1) nach einem der Ansprüche 1 bis 3, wobei die Pumpe (1) dazu eingerichtet ist, Pumpendaten nutzerbasiert und/oder eventbasiert auf der Komponente (2) zur kontaktlosen Datenübertragung zu speichern.

5. Medizinische Pumpe (1) nach einem der Ansprüche 1 bis 4, wobei die Pumpendaten ausgewählt sind aus der Gruppe umfassend Fehlerdaten, Fehleranalysedaten, Gerätestatusdaten, Gerätetypdaten, Leistungsdaten, Zeitstempel, Eingabeprotokolle, Alarme und Informationen zu einem verwendeten Einmalartikel.

6. Medizinische Pumpe (1) nach einem der Ansprüche 1 bis 5, wobei die Komponente (2) zur kontaktlosen Datenübertragung durch wenigstens ein Sicherheitsmerkmal vor einem unauthorisierten Auslesen der Pumpendaten mittels des mobilen Endgeräts (3) geschützt ist.

7. Medizinische Pumpe (1) nach einem der Ansprüche 1 bis 6, wobei die Komponente (2) zur kontaktlosen Datenübertragung in einem vor Störungen abgeschirmten Bereich der medizinischen Pumpe (1) angeordnet ist.

8. Medizinische Pumpe (1) nach einem der Ansprüche 1 bis 7, wobei die Datenübertragung von Pumpendaten von der Komponente (2) zur kontaktlosen Datenübertragung an ein mobiles Endgerät (3) von einer Internetverbindung der Pumpe (1) unabhängig ist.

9. Verfahren zur kabellosen Übertragung von Pumpendaten, umfassend:
- einen Schritt a) des Bereitstellens einer medizinischen Pumpe (1), bevorzugt nach einem der Ansprüche 1 bis 8, mit einer Komponente (2) zur kontaktlosen Datenübertragung,
- einen Schritt b) des Übertragens von Pumpendaten der medizinischen Pumpe (1) an ein mobiles Endgerät (3) mittels der Komponente (2) zur kontaktlosen Datenübertragung,
- einen Schritt c) des Übertragens von in Schritt b) ausgelesenen Pumpendaten von dem mobilen Endgerät (3) an eine Serviceeinrichtung (4),
wobei die Pumpendaten in einem für die Übertragung oder Speicherung geeigneten Datenpaket für die Verarbeitung und Analyse durch nachgelagerte Systeme oder Anwendungen, insbesondere eine Serviceeinrichtung (4), strukturiert sind und weiter bevorzgut das Datenpaket einen zeitlichen Ausschnitt um ein Ereignis und/oder bestimmte Messwerte, die sich auf Komponenten wie den Antrieb oder die Elektronik beziehen, ausgewählt ist.

10. Verfahren nach Anspruch 9, weiter umfassend:
- einen Schritt c) des Übertragens der in Schritt b) ausgelesenen Pumpendaten von dem mobilen Endgerät (3) an eine Serviceeinrichtung (4), wobei bevorzugt die Übertragung der Pumpendaten an die Serviceeinrichtung (4) in einem Schritt c) verschlüsselt erfolgt.

11. App, bevorzugt auf einem mobilen Endgerät (3), dazu eingerichtet
- von einer Komponente (2) zur kontaktlosen Datenübertragung einer medizinischen Pumpe (1) an das mobile Endgerät (3) übertragene Pumpendaten darzustellen und/oder zu analysieren, und/oder
- von einer Komponente (2) zur kontaktlosen Datenübertragung einer medizinischen Pumpe (1) an das mobile Endgerät (3) übertragene Pumpendaten an eine Serviceeinrichtung (4) für die Verarbeitung und Analyse der Pumpendaten zu übertragen.

12. App nach Anspruch 11, wobei die App dazu eingerichtet ist, auf dem mobilen Endgerät die ausgelesenen Daten zu filtern und/oder zu sortieren.

13. App nach Anspruch 11 oder 12, wobei die App dazu eingerichtet ist, eine Benachrichtigung an den Benutzer zu senden, sobald die Daten erfolgreich übertragen wurden und/oder einen benutzerdefinierten Kommentar zu den übertragenen Daten hinzuzufügen und zu übertragen.

14. App nach einem der Ansprüche 11 bis 13, wobei die App eine Schnittstelle zur Integration mit anderen Krankenhausinformationssystemen aufweist.

15. System zur kabellosen Übertragung von Pumpendaten, umfassend:
- eine medizinische Pumpe (1) nach einem der Ansprüche 1 bis 8, umfassend eine Komponente (2) zur kontaktlosen Datenübertragung von Pumpendaten an ein mobiles Endgerät (3), wobei die Komponente (2) die Pumpendaten umfasst,
- ein mobiles Endgerät (3), das als Reader für die Pumpendaten zur kontaktlosen Datenübertragung der Komponente (2) zur kontaktlosen Datenübertragung an das mobiles Endgerät (3) dient,
- eine App nach Anspruch 14, und
- eine Serviceeinrichtung (4), die Pumpendaten von dem mobilen Endgerät (3) empfängt und analysiert.
